# EUROPEAN PATENT APPLICATION

(11) **EP 4 014 884 A1**
(43) Date of publication of application: **22.06.2022**
(21) Application number: 20214799.7
(22) Date of filing: 17.12.2020
(51) Int. Cl.: A61B 8/08

(54) **APPARATUS FOR USE IN ANALYSING AN ULTRASOUND IMAGE OF A SUBJECT**

(71) Applicant: Koninklijke Philips N.V., 5656 AG Eindhoven (NL)
(72) Inventor: ORASANU, Eliza Teodora, 5656 AE Eindhoven (NL); SCHMIDT-RICHBERG, Alexander, 5656 AE Eindhoven (NL); FRANZ, Astrid Ruth, 5656 AE Eindhoven (NL); LORENZ, Cristian, 5656 AE Eindhoven (NL)
(74) Representative: Philips Intellectual Property & Standards

(57) **Abstract**

An apparatus for use in analysing an ultrasound image of a subject comprises a memory comprising instruction data representing a set of instructions, and a processor configured to communicate with the memory and to execute the set of instructions. The set of instructions, when executed by the processor, cause the processor to: obtain a measurement of a distance between anatomical features of interest in the ultrasound image of the subject, compare the obtained measurement to a predefined criterion for normal distances between the anatomical features of interest, and if the obtained measurement is abnormal when compared to the predefined criterion, send an instruction to a display to cause the display to render the ultrasound image of the subject and further render an indication of the abnormality.

## Description

### FIELD OF THE INVENTION

The disclosure herein relates to ultrasound systems. Particularly but non-exclusively, the disclosure relates to methods, apparatus and ultrasound systems for use in analysing an ultrasound image of a subject.

### BACKGROUND OF THE INVENTION

Ultrasound (US) is the modality of choice for fetal screening as it is able to render fetal anatomy in sufficient detail, while at the same being time and cost effective with no known adverse effects. During an ultrasound examination, fetal growth is assessed and the sonographer/clinician studies the obtained images for abnormalities. Detailed examinations of this type typically start at 18 to 22 weeks gestational age (GA) where specific standardised measurements are recommended.

With respect to the face, it is preferable to systematically examine the fetal face in three different planes to properly assess the facial structures, as is described in the paper by Annisa Shui, Lam Mak and Kwok Yin Leung entitled: "Prenatal ultrasonography of craniofacial abnormalities", Ultrasonography 38(1):13-24, January 2019.

In the context of fetal facial examinations, 3D ultrasound provides a various advantages over 2D since 2D is dependent on the user's ability to capture the correct view/slice for the examination. Since coronal, sagittal and transverse views are all investigated during a facial abnormality assessment, 3D ultrasound captures an overall view of the face, thus minimizing the risk of misdiagnosis. Furthermore, fetal 3D facial rendering is generally in high demand, and thus widely available in ultrasound machines.

More widely, ultrasound is used in a similar manner to look for abnormalities in children and adults. For example, abnormalities of the heart and/or liver.

### SUMMARY OF THE INVENTION

As noted above, ultrasound examinations are used to perform screening examinations for children and adults, for instance fetal screening examinations. In such examinations, screening for abnormalities is performed by the examining sonographer. For example, in fetal examinations, a sonographer may look for facial abnormalities such as facial cleft, cataract, microcephaly, or facial asymmetries which are associated with several abnormalities, conditions and genetic syndromes. It is important to determine if any of them are present for future intervention, diagnosis and/or treatment. While 3D rendering in ultrasound examinations is becoming more commonplace, this is mostly done qualitatively. It is an object of the disclosure herein to improve on this situation and provide improved methods, apparatuses and systems for assessing ultrasound images of anatomical features of interest of a subject.

According to a first aspect, there is provided an apparatus for use in analysing an ultrasound image of a subject. The apparatus comprises a memory comprising instruction data representing a set of instructions, and a processor configured to communicate with the memory and to execute the set of instructions. The set of instructions, when executed by the processor, cause the processor to: obtain a measurement of a distance between anatomical features of interest in the ultrasound image of the subject, compare the obtained measurement to a predefined criterion for normal distances between the anatomical features of interest, and if the obtained measurement is abnormal when compared to the predefined criterion, send an instruction to a display to cause the display to render the ultrasound image of the subject and further render an indication of the abnormality.

According to a second aspect there is an ultrasound system comprising the apparatus of the first aspect and further comprising a transducer for obtaining the ultrasound image of the subject. The system may further comprise the display.

According to a third aspect there is a computer implemented method for use in analysing an ultrasound image of a subject. The method comprises obtaining a measurement of a distance between anatomical features of interest in the image of the subject, comparing the obtained measurement to a predefined criterion for normal distances between the anatomical features of interest, and if the obtained measurement is abnormal when compared to the predefined criterion, sending an instruction to a display to cause the display to render the image of the subject and further render an indication of the abnormality.

According to a fourth aspect there is a computer program product comprising a computer readable medium, the computer readable medium having computer readable code embodied therein, the computer readable code being configured such that, on execution by a suitable computer or processor, the computer or processor is caused to perform the method of the third aspect.

Thus, embodiments herein provide effective quantitative evaluation of features of interest, so as to provide the sonographer/clinician with quick suggestions of regions that may require further attention and closer investigation. Generally, in embodiments described below, landmark detection as well as the distances between them can be implemented so that a quantitative visualization may be added on top of the qualitative reconstruction of an ultrasound image. Embodiments herein thus enable a more quantitative and standardised approach to ultrasound anomaly examinations, helping the sonographer to reduce errors and improve screening.

These and other aspects will be apparent from and elucidated with reference to the embodiment(s) described hereinafter.

### BRIEF DESCRIPTION OF THE DRAWINGS

Example embodiments will now be described, by way of example only, with reference to the following drawings, in which:
Fig. 1 shows an apparatus according to some embodiments herein;
Fig. 2 shows an ultrasound system according to some embodiments herein;
Fig. 3 shows a method according to some embodiments herein;
Fig. 4a shows an example ultrasound image of a face of a fetus; and
Fig. 4b shows example highlighting according to an embodiment herein.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail.

As noted above, embodiments herein aim to improve ultrasound screening for anomalies in areas such as, for example, fetal monitoring, and/or heart monitoring and/or liver monitoring in children and adults. Although the present description mainly refers to foetus, heart and liver monitoring, it is to be understood that the concepts herein described also apply to ultrasound image(s) of other region and/or areas of interests of a living entity.

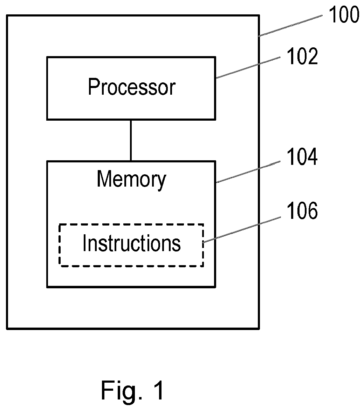

Fig. 1, shows an apparatus 100 for use in analysing an ultrasound image or a sequence of ultrasound images of a subject, according to some embodiments herein. Generally, the apparatus may form part of a computer apparatus or system e.g. such as a laptop, desktop computer or other computing device. In some embodiments, the apparatus 100 may form part of a distributed computing arrangement or the cloud.

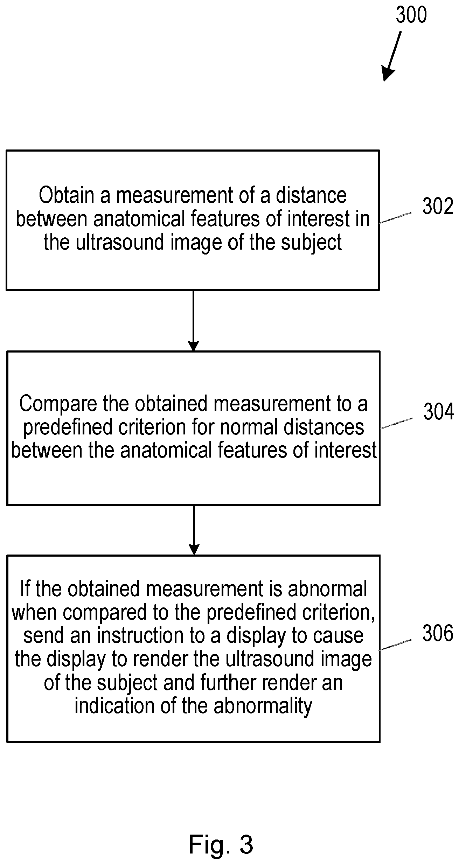

Generally, the apparatus may be adapted or configured to perform any of the embodiments of the methods described herein, such as the method 300 as described below with respect to Fig. 3.

The apparatus comprises a memory 104 comprising instruction data representing a set of instructions 106 and a processor 102 (e.g. processing circuitry or logic) configured to communicate with the memory and to execute the set of instructions 106. Briefly, the set of instructions 106, when executed by the processor 102, cause the processor 102 to obtain a measurement of a distance between anatomical features of interest in the ultrasound image or a sequence of ultrasound images of the subject, compare the obtained measurement to a predefined criterion for normal distances between the anatomical features of interest, and if the obtained measurement is abnormal when compared to the predefined criterion, send an instruction to a display to cause the display to render the ultrasound image or a sequence of ultrasound images of the subject and further render an indication of the abnormality.

As described above, current ultrasound screening examinations tend to rely on the expertise of the sonographer to visually recognise abnormalities and/or make measurements on the screen in order to determine the presence of an anomaly. The proposed apparatus herein automates this process to provide quantifiable measures that can be highlighted to the sonographer, thus helping the sonographer to quickly recognise and diagnose abnormalities in a subject, such as for example, fetal abnormalities.

In more detail, the processor 102 may comprise one or more processors, processing units, multi-core processors or modules that are configured or programmed to control the apparatus 100 in the manner described herein. In particular implementations, the processor 102 can comprise a plurality of software and/or hardware modules that are each configured to perform, or are for performing, individual or multiple steps of the method described herein. The processor 102 can comprise one or more processors, processing units, multi-core processors and/or modules that are configured or programmed to control the apparatus 100 in the manner described herein. In some implementations, for example, the processor 102 may comprise a plurality of (for example, interoperated) processors, processing units, multi-core processors and/or modules configured for distributed processing. It will be appreciated by a person skilled in the art that such processors, processing units, multi-core processors and/or modules may be located in different locations and may perform different steps and/or different parts of a single step of the method described herein.

The memory 104 is configured to store program code that can be executed by the processor 102 to perform the method described herein. Alternatively or in addition, one or more memories 104 may be external to (i.e. separate to or remote from) the apparatus 100. For example, one or more memories 104 may be part of another device. Memory 104 can be used to store the ultrasound image, the obtained measurements and/or any other information or data received, calculated or determined by the processor 102 of the apparatus 100 or from any interfaces, memories or devices that are external to the apparatus 100. The processor 102 may be configured to control the memory 104 to store the ultrasound image, the obtained measurements and/or the any other information or data received, calculated or determined by the processor.

In some embodiments, the memory 104 may comprise a plurality of sub-memories, each sub-memory being capable of storing a piece of instruction data. For example, at least one sub-memory may store instruction data representing at least one instruction of the set of instructions, while at least one other sub-memory may store instruction data representing at least one other instruction of the set of instructions.

It will be appreciated that Fig. 1 only shows the components required to illustrate this aspect of the disclosure and, in a practical implementation, the apparatus 100 may comprise additional components to those shown. For example, the apparatus 100 may further comprise a display. A display may comprise, for example, a computer screen, and/or a screen on a mobile phone or tablet. The apparatus may further comprise a user input device, such as a keyboard, mouse or other input device that enables a user to interact with the apparatus, for example, to provide initial input parameters to be used in the method described herein. The apparatus 100 may comprise a battery or other power supply for powering the apparatus 100 or means for connecting the apparatus 100 to a mains power supply.

In some embodiments, the apparatus 100 may be comprised in an ultrasound system. For example, an ultrasound system may comprise the apparatus 100 described above, and further comprise a transducer with which to record the ultrasound image data.

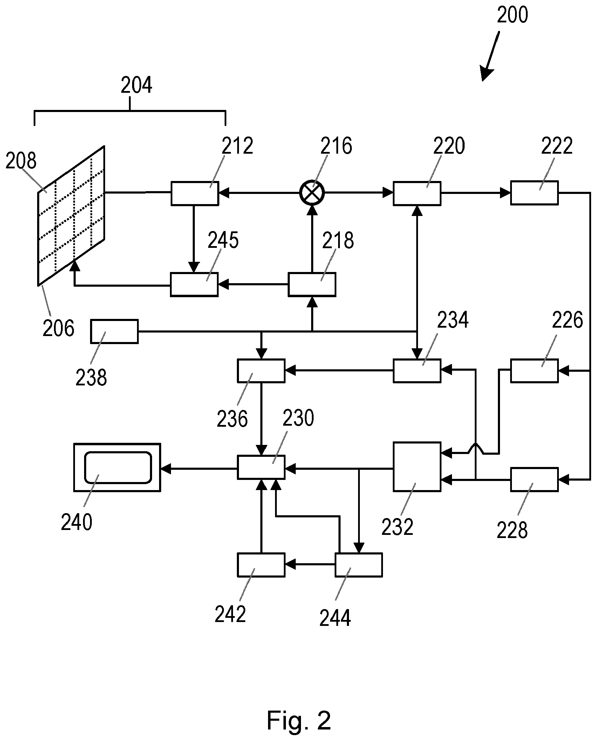

Fig. 2 shows an example ultrasound system 200. The ultrasound system 200 may comprise the apparatus 100 described above. In other embodiments, components of the ultrasound system 200 may be adapted to perform the method 300 described below.

The system 200 comprises an array transducer probe 204 which has a transducer array 206 for transmitting ultrasound waves and receiving echo information. The transducer array 206 may comprise CMUT transducers; piezoelectric transducers, formed of materials such as PZT or PVDF; or any other suitable transducer technology. In this example, the transducer array 206 is a two-dimensional array of transducers 208 capable of scanning either a 2D plane or a three dimensional volume of a region of interest. In another example, the transducer array may be a ID array.

The transducer array 206 may be coupled to a microbeamformer 212 which controls reception of signals by the transducer elements. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

In an alternative embodiment, instead of a microbeamformer 212, the transducer array may be operated directly by a main system beamformer (not shown in Fig. 2).

The system 200 may further comprise a transmit/receive (T/R) switch 216, which the microbeamformer 212 can be coupled to and which switches the array between transmission and reception modes. The transmission of ultrasound beams from the transducer array 206 is directed by a transducer controller 218 coupled to the microbeamformer by the T/R switch 216 and a main transmission beamformer (not shown), which can receive input from the user's operation of the user interface or control panel 238. The controller 218 can include transmission circuitry arranged to drive the transducer elements of the array 206 (either directly or via a microbeamformer) during the transmission mode.

It is noted that in an alternative embodiment, where instead of a microbeamformer 212, the transducer array is operated directly by a main system beamformer, a T/R switch 216 may protect the main beamformer 220 from high energy transmit signals.

In a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one dimensional line of transducers or a two dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal represent echoes from structures at increasing depths within the subject.

One of the functions controlled by the transducer controller 218 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 218 can be coupled to control a DC bias control 245 for the transducer array. The DC bias control 245 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 212 and are then passed to a main receive beamformer 220 where the partially beamformed signals from individual patches of transducers are combined into a fully beamformed signal, referred to as radio frequency (RF) data. The beamforming performed at each stage may be carried out as described above, or may include additional functions. For example, the main beamformer 220 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 222. The signal processor 222 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Fig. 2 only the receiver beamformers 212, 220 are shown, for simplicity. In the complete system, there will also be a transmission chain with a transmission micro beamformer, and a main transmission beamformer.

The function of the micro beamformer 212 is to provide an initial combination of signals in order to decrease the number of analog signal paths. This is typically performed in the analog domain.

The final beamforming is done in the main beamformer 220 and is typically after digitization.

The transmission and reception channels use the same transducer array 206 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 226 and a Doppler processor 228. The B mode processor 226 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 228 processes temporally distinct signals arising from tissue movement and blood flow for the detection of moving substances, such as the flow of blood cells in the image field. The Doppler processor 228 typically includes a wall filter with parameters set to pass or reject echoes returned from selected types of materials in the body.

The structural and motion signals produced by the B mode and Doppler processors are coupled to a scan converter 232 and a multi-planar reformatter 244. The scan converter 232 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 240. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image is able to depict tissue motion and blood flow within the structural image field. The multi-planar reformatter will convert echoes that are received from points in a common plane in a volumetric region of the body into an ultrasound image of that plane, as described in US Pat. 6,443,896 (Detmer). A volume renderer 242 converts the echo signals of a 3D data set into a projected 3D image as viewed from a given reference point as described in US Pat. 6,530,885 (Entrekin et al.).

The 2D or 3D images are coupled from the scan converter 232, multi-planar reformatter 244, and volume renderer 242 to an image processor 230 for further enhancement, buffering and temporary storage for display on an image display 240. The imaging processor may be adapted to remove certain imaging artifacts from the final ultrasound image, such as for example,: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 228 and tissue structure information produced by the B mode processor 226 are coupled to a quantification processor 234. The quantification processor may be used for making measurements in the images. The quantification processor may receive input from a user control panel 238, as described in detail below.

Output data from the quantification processor is coupled to a graphics processor 236 for the reproduction of measurement graphics and values with the image on the display 240, and for audio output from the display device 240. The graphics processor 236 can also generate graphic overlays for display with the ultrasound images. These graphic overlays can contain standard identifying information such as patient name, date and time of the image, imaging parameters, and the like. In the context of the disclosure herein the graphic overlays may comprise highlighting of regions likely to contain an abnormality, as described below.

For these purposes the graphics processor receives input from the user interface 238, such as patient name. The user interface is also coupled to the transmit controller 218 to control the generation of ultrasound signals from the transducer array 206 and hence the images produced by the transducer array and the ultrasound system. The transmit control function of the controller 218 is only one of the functions performed. The controller 218 also takes account of the mode of operation (given by the user) and the corresponding required transmitter configuration and band-pass configuration in the receiver analog to digital converter. The controller 218 can be a state machine with fixed states.

The user interface is also coupled to the multi-planar reformatter 244 for selection and control of the planes of multiple multi-planar reformatted (MPR) images which may be used to perform quantified measures in the image field of the MPR images.

The skilled person will appreciate that the detail provided above and the components illustrated in Fig. 2 are an example only and that an ultrasound system may have different components to those illustrated therein.

Turning now back to Fig. 1, as noted above, the apparatus 100 is generally for analysing an ultrasound image or a sequence of ultrasound images of a subject. For example, an image such as that obtained in an ultrasound examination (otherwise known as an ultrasound screening).

In some embodiments herein, the subject may be a fetus. In other embodiments, the subject may be a born mammal, such as a human adult or a human child.

Generally, the operations described herein may be performed in real-time, for example, during a "live" or ongoing examination. Alternatively, the operations herein may be performed on historical data, e.g. ultrasound images that were obtained as part of a concluded or previous examination.

In some embodiments, the processor may be caused to obtain the ultrasound image of the subject. This may involve obtaining raw ultrasound data (e.g. RF data as described above). Thus in some embodiments, the processor may also be caused to process RF data to obtain the ultrasound image. The skilled person will be familiar with image processing techniques for processing RF data to obtain an ultrasound image. As noted above, raw RF data may be obtained in real-time during a live examination, in which case the apparatus may be configured to receive the RF data from a transducer (for example, using a wired or wireless connection).

In other embodiments, the processor may obtain the ultrasound image from a database of ultrasound images.

Generally, the ultrasound image may be two-dimensional (e.g. an ultrasound image frame) or three-dimensional. The ultrasound image may have been obtained using an imaging mode of an ultrasound system, for example a B-mode imaging mode.

As described above, the processor is caused to obtain a measurement of a distance between anatomical features of interest in the ultrasound image of the subject (e.g. an adult, child or fetus; otherwise described as the patient or subject of the ultrasound examination).

The ultrasound image may comprise an image of a region of interest (ROI) of (e.g. within or on the surface of) the subject. For example, a ROI may comprise the subject's face, the subject's liver, the subject's hips, or the subject's heart. The skilled person will appreciate that these are merely examples and that the region of interest may comprise other anatomical features. Generally, the anatomical features of interest described herein may be landmarks of a or on a region of interest.

In examples where the subject is a fetus (and e.g. the ultrasound image comprises an image of a fetus), anatomical features of interest may comprise, for example, features on the face of the fetus. The face of the fetus may thus be a ROI. For example, the ultrasound image may comprise an ultrasound image of the face of the fetus and the anatomical features of interest may comprise landmarks on the face of the fetus. Examples of landmarks on the face include, for example, the nasion, eyebrows, forehead, and chin. Other example anatomical features of interest on the face of a fetus include for example, the landmarks: PN - pronasal, SN - subnasal, ALA - alae, EN - endocanthionis, N - nasion, IE-inner eyebrow, CH - chelion, LS - labrum superior, and LI - labrum inferior. These are described and illustrated in the paper by E. Vezzetti, D. Speranza, F. Marcolin, et al. entitled "Exploiting 3D Ultrasound for fetal diagnosis purpose through facial landmarking", Acta Stereol 33(3): 167-188, 2014.

The skilled person will appreciate that this is merely an example however and that the apparatus and methods herein may be used to analyse ultrasound images of other anatomical features of interest. For example, including but not limited to, ultrasound images of the fetal heart. In other words, a ROI may comprise the fetal heart. In this example, landmarks may include, for example, the right and left atrium, right and left ventricle, aorta or pulmonary tract. These are described in the paper in the paper by Picazo-Angelin et al. 2018 entitled "Anatomy of the normal fetal heart: The basis for understanding fetal echocardiography*.*

In another example, the ultrasound image comprises an ultrasound image of the hips of a fetus. In other words, a ROI may comprise the hips of the fetus. In this example, the anatomical features of interest may comprise, for example, the iliac crests landmarks, as described by Lee et al. 2010 in the paper entitled: "Iliac Crest Angle: A Novel Sonographic Parameter for the Prediction of Down Syndrome Risk During the Second Trimester of Pregnancy".

In another example, the ultrasound image comprises an image of the brain of a fetus. In other words, a ROI may comprise the brain of the fetus. In this example the anatomical features may comprise, for example, the left and right ventricle, and/or the cave of septum pellucidum. In other examples, ultrasound images of other areas of a fetal vascular system may be obtained.

This method is further not limited to ultrasound images of a fetus, but can also be used to detect abnormalities in children and adults. For example, in heart abnormality detection (e.g. using the same anatomical features/landmarks as described above for the fetal heart). In other examples, the apparatus and methods described herein may be used to detect abnormalities of the liver or hips in adults and children.

The processor may be caused to use landmark detection to locate the anatomical features of interest in the ultrasound image of the subject. In some embodiments, the processor is caused to perform the landmark detection using a model trained using a machine learning process (e.g. trained to label landmarks in an image).

Landmark detection may be performed, for example, using classic machine learning or AI-based algorithms e.g. such as neural networks. The skilled person will be familiar with machine learning and methods in which a machine learning model such as a neural network may be trained (e.g. via logistic regression and/or back propagation) to label an image based on a training data set containing labelled examples. The skilled person will appreciate that neural networks are given as an example and that other machine learning models may also be used to perform landmark detection of anatomical features of interest in ultrasound images of a subject, for example, autoencoder networks or generative adversarial networks. As an example, landmark detection using a neural network is described, for example, in the paper by Schmidt-Richberg, A, et al.: Proc. SPIE (2019).

In three-dimensional ultrasound images, machine learning models may exploit the full 3D information, which can improve the robustness of the method. For example, some landmarks may be partially in a shadowed region or the sonographer/user might not have sufficient experience to capture an optimal view with which to define them correctly (e.g. when viewing in two-dimensions). Thus, a machine learning model trained to perform landmark detection on image information extracted from neighbouring slices of a three-dimensional image can improve the accuracy of the landmark detection.

In this way, the apparatus herein can make measurements of landmarks, and thus highlight potential anomalies to the user/sonographer in a three-dimensional ultrasound image volume, even if the user is not observing an optimal surface view or reconstruction of the three dimensional volume. This can be particularly useful to avoid missing certain diagnosis.

Once the locations of the anatomical features of interest have been determined, a measurement can be made e.g. between pairs of anatomical features of interest (e.g. between pairs of landmarks/points in the image). The measurement may be calculated, for example, in the same manner as is performed by known measurement tools such as calipers that can be placed in an image.

The processor is then caused to compare the obtained measurement to a predefined criterion (or set of criteria) for normal distances between the anatomical features of interest. Put another way, distances between landmarks may be computed and compared to the known-norm of these measures.

For example, the predefined criterion may comprise a "normal" (e.g. average) value for the distance between the anatomical features of interest. For example, such a normal value may be determined from a reference population of subjects (e.g. using data from healthy subjects), from academic literature or other well-defined studies. Examples of normal measurements that may form the basis of the predefined criterion described herein are given in the paper by A. T. Papageorghiou, et al. entitled: "International standards for fetal growth based on serial ultrasound measurements: the Fetal Growth Longitudinal Study of the INTERGROWTH-21st Project", The Lancet 384, pp. 869-879, 2014.

The predefined criterion may further comprise a threshold deviation from the normal/average value that encompasses (or is representative of) healthy deviations from the normal/average value as observed in subject populations. In another example, the predefined criterion may comprise a range of values within which the measurement may be considered normal.

Generally, normal is used herein to denote a healthy value for which further medical follow up is not (or unlikely to be) required. In embodiments where the subject is a fetus, normal may be used to indicate a value consistent with a fetus not exhibiting a fetal abnormality. Generally, the predefined criterion may be dependent on the age of the subject (or gestational age of the fetus) and/or other factors that may affect the measurement, such as for example, ethnicity of the subject.

Thus the processor is caused to compare the measured value to the predefined criterion (or criteria) and if the obtained measurement is inconsistent with the predefined criterion, the measurement is flagged as an anomaly. In embodiments where the ultrasound image is an image of a fetus, if the obtained measurement is inconsistent with the predefined criterion, this may potentially be indicative of a fetal abnormality.

If the obtained measurement is abnormal when compared to the predefined criterion, the processor is then caused to send an instruction to a display to cause the display to render the ultrasound image of the subject and further render an indication of the abnormality.

As an example, in an embodiment where the predefined criterion comprises a normal value for the distance between the anatomical features of interest, as determined from a reference population of subjects, the obtained measurement may be abnormal if the measurement deviates from the normal value by a threshold amount. Such a threshold may comprise a standard deviation, for example, or any other statistical measure of deviation from the population norm.

The display may be caused to display/render the ultrasound image of the subject (or render the ROI of the subject) in any known manner. For example, for an ultrasound image of the face of a fetus, a 3D fetal face model may be extracted which can then be used to create a 3D rendering of the face. This task may be performed, for example, using histograms, thresholding, 3D models etc. See for example, the paper by L. Nonacina et. al, entitled "Automatic 3D foetal face model extraction from ultrasonography through histogram processing"; Journal of Medical Ultrasound, 24[4]: 142-149, December 2016.

The display may then be caused to render the indication of the abnormality over the image of the subject. For example, the instruction may cause the display to highlight a region comprising the abnormality in the ultrasound image of the subject. For example, the region comprising the abnormality may be highlighted in a different colour or texture in the ultrasound image of the subject.

In some embodiments, the instruction is to cause the display to indicate positions of the anatomical features of interest on the rendered ultrasound image of the subject. For example, the display may render the locations of any landmarks determined, as described above.

The instruction may be sent by the processor to cause the display to indicate the distance between the anatomical features of interest. For example, the display may display the distance (e.g. in alpha numeric characters). As another example, the instruction may be to cause the display to indicate the distance by means of a caliper between the positions of the anatomical features on the rendered ultrasound image of the subject. A caliper may comprise, for example, a line drawn between the positions of the anatomical features.

As an example, measured distances may be displayed on a 3D rendering of a fetal face, identifying regions with potential abnormalities that require further investigation. If the distance between the eyebrows does not fit into the usual expected range for the fetus' gestational age, it can be highlighted to the sonographer for investigation.

In this way, distances between landmarks may be represented on a 2D or 3D rendering, highlighting the regions that may present abnormalities and need further investigation. As noted above, the method herein may be of particular benefit in 3D to highlight potential anomalies to the user/sonographer in a three-dimensional volume, even if the user is not observing an optimal reconstruction or view of said volume.

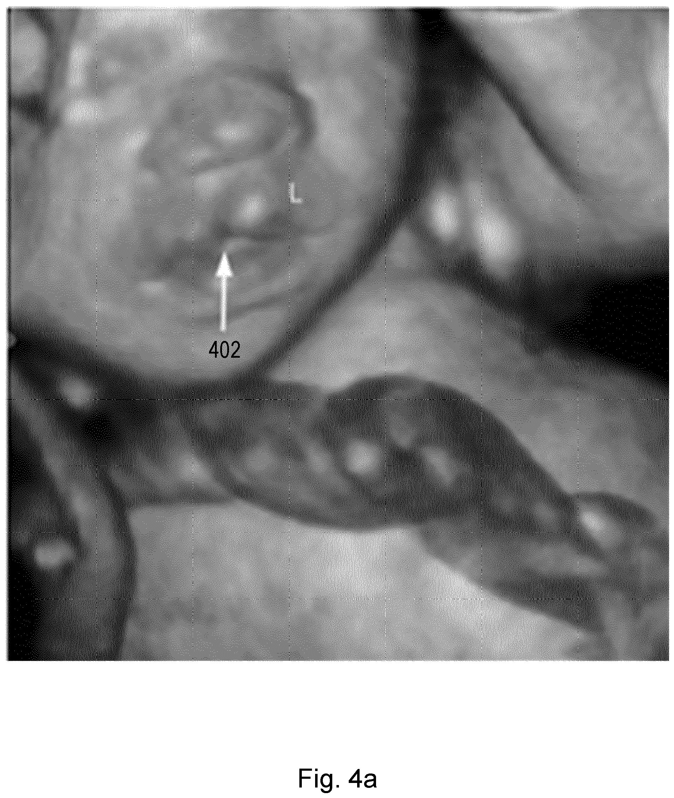

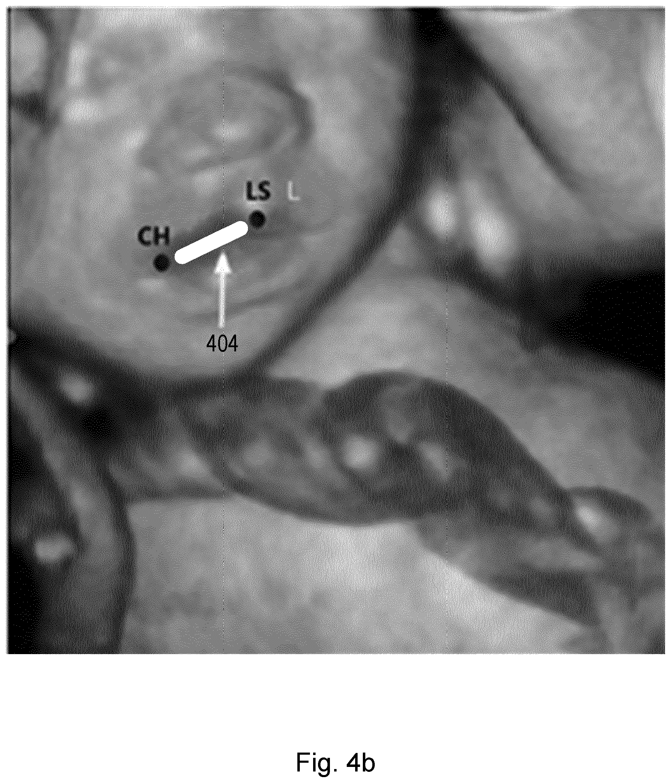

An example is given in Figs. 4a and 4b. Fig. 4a illustrates a three-dimensional rendering of a second trimester fetus with a unilateral cleft lip 402. Fig. 4b shows the same image with overlay to highlight the abnormality. In this example, the positions of the facial landmarks Chelion (CH) and Labrum Superior (LS) are marked and the surface distance difference between 2 extracted landmarks is highlighted 404, which is different to a known norm. In this way, the abnormal measurement is highlighted to the sonographer for follow up.

Turning to Fig. 3, there is a computer implemented method for use in analysing an ultrasound image of a subject. Briefly, in a first step, the method comprises obtaining 302 a measurement of a distance between anatomical features of interest in the image of the subject. In a second step the method comprises comparing 304 the obtained measurement to a predefined criterion for normal distances between the anatomical features of interest. In a third step, if the obtained measurement is abnormal when compared to the predefined criterion, the method then comprises sending 306 an instruction to a display to cause the display to render the image of the subject and further render an indication of the abnormality.

Obtaining a measurement of a distance between anatomical features of interest in the image of the subject was described in detail above with respect to the apparatus 100 and the detail therein will be understood to apply equally to embodiments of the method 300.

Comparing the obtained measurement to a predefined criterion for normal distances between the anatomical features of interest was described in detail above with respect to the apparatus 100 and the detail therein will be understood to apply equally to embodiments of the method 300.

A step of, if the obtained measurement is abnormal when compared to the predefined criterion, sending an instruction to a display to cause the display to render the image of the subject and further render an indication of the abnormality was described in detail above with respect to the apparatus 100 and the detail therein will be understood to apply equally to embodiments of the method 300.

There is thus provided a method of highlighting possible abnormalities in an ultrasound image of a subject to a sonographer in a quick and reliable manner for follow up.

In another embodiment, there is provided a computer program product comprising a computer readable medium, the computer readable medium having computer readable code embodied therein, the computer readable code being configured such that, on execution by a suitable computer or processor, the computer or processor is caused to perform the method or methods described herein.

Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An apparatus for use in analysing an ultrasound image of a subject, the apparatus comprising:
a memory comprising instruction data representing a set of instructions; and
a processor configured to communicate with the memory and to execute the set of instructions, wherein the set of instructions, when executed by the processor, cause the processor to:
obtain a measurement of a distance between anatomical features of interest in the ultrasound image of the subject;
compare the obtained measurement to a predefined criterion for normal distances between the anatomical features of interest; and
if the obtained measurement is abnormal when compared to the predefined criterion, send an instruction to a display to cause the display to render the ultrasound image of the subject and further render an indication of the abnormality.

2. An apparatus as in claim 1 wherein the instruction is to cause the display to highlight a region comprising the abnormality in the ultrasound image of the subject.

3. An apparatus as in claim 2 wherein the instruction is to cause the region comprising the abnormality to be highlighted in a different colour or texture in the ultrasound image of the subject.

4. An apparatus as in claim 1, 2 or 3 wherein the instruction is to cause the display to indicate positions of the anatomical features of interest on the rendered ultrasound image of the subject.

5. An apparatus as in claim 4 wherein the instruction is to further cause the display to indicate the distance between the anatomical features of interest.

6. An apparatus as in claim 5 wherein the instruction is to cause the display to indicate the distance by means of a caliper between the positions of the anatomical features on the rendered ultrasound image of the subject.

7. An apparatus as in any one of the preceding claims wherein the processor being caused to obtain the measurement of the distance between the anatomical features of interest comprises the processor being caused to:
use landmark detection to locate the anatomical features of interest in the ultrasound image of the subject.

8. An apparatus as in claim 7 wherein the processor is caused to perform the landmark detection using a model trained using a machine learning process.

9. An apparatus as in any one of the preceding claims wherein the predefined criterion comprises a normal value for the distance between the anatomical features of interest as determined from a reference population of subjects; and
wherein the obtained measurement is abnormal if the measurement deviates from the normal value by a threshold amount.

10. An apparatus as in any one of the preceding claims wherein the ultrasound image comprises an image of a region of interest (ROI) of the subject and the anatomical features of interest comprise landmarks on said region of interest.

11. An apparatus as in claim 10 wherein the subject is a fetus, the ROI comprises the face of the fetus and the anatomical features of interest comprise landmarks on the face of the fetus.

12. An apparatus as in any one of the preceding claims wherein the ultrasound image comprises a three-dimensional ultrasound image.

13. An ultrasound system comprising the apparatus of any one of the preceding claims, further comprising:
a transducer for obtaining the ultrasound image of the subject; and
the display.

14. A computer implemented method for use in analysing an ultrasound image of a subject, the method comprising:
obtaining a measurement of a distance between anatomical features of interest in the image of the subject;
comparing the obtained measurement to a predefined criterion for normal distances between the anatomical features of interest; and
if the obtained measurement is abnormal when compared to the predefined criterion, sending an instruction to a display to cause the display to render the image of the subject and further render an indication of the abnormality.

15. A computer program product comprising a computer readable medium, the computer readable medium having computer readable code embodied therein, the computer readable code being configured such that, on execution by a suitable computer or processor, the computer or processor is caused to perform the method as claimed in claim 14.
